(19) Europäisches Patentamt
European Patent Office
Office européen des brevets

(11) Veröffentlichungsnummer: **0 017 931**
**B1**

(12) **EUROPÄISCHE PATENTSCHRIFT**

(45) Veröffentlichungstag der Patentschrift: **07.03.84**

(51) Int. Cl.³: **C 07 D 265/20**, C 07 D 265/22, A 01 N 43/86

(21) Anmeldenummer: **80101957.1**

(22) Anmeldetag: **11.04.80**

(54) **4H-3,1-Benzoxazinderivate, Verfahren zu ihrer Herstellung und ihre Verwendung zur Bekämpfung unerwünschten Pflanzenwuchses.**

(30) Priorität: **12.04.79 DE 2914915**

(43) Veröffentlichungstag der Anmeldung: **29.10.80 Patentblatt 80/22**

(45) Bekanntmachung des Hinweises auf die Patenterteilung: **07.03.84 Patentblatt 84/10**

(84) Benannte Vertragsstaaten: **AT BE CH DE FR GB IT LI LU NL SE**

(56) Entgegenhaltungen:
**BE - A - 648 259**
**DE - A - 1 670 375**
**DE - A - 2 241 012**
**DE - A - 2 556 590**
**FR - A - 2 121 341**
**US - A - 3 357 977**
**US - A - 3 914 121**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Wuerzer, Bruno, Dr. Dipl.-Landwirt, Ruedigerstrasse 13, D-6701 Otterstadt (DE)**

**EP 0 017 931 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4H-3,1-Benzoxazinderivate, Verfahren zu ihrer Herstellung und ihre Verwendung zur Bekämpfung unerwünschten Pflanzenwuchses

Die vorliegende Erfindung betrifft 4H-3,1-Benzoxazinderivate, ein Verfahren zu ihrer Herstellung, Herbizide, die diese Verbindungen als Wirkstoffe enthalten, und Verfahren zur Bekämpfung unerwünschten Pflanzenwuchses mit diesen Verbindungen.

Substituierte 4H-3,1-Benzoxazin-4-one sind bekannt als Zwischenprodukte für die Synthese pharmakologisch wirksamer Verbindungen (DE-A-1 670 375, DE-A-2 556 590) oder als herbizide Wirkstoffe, wobei insbesondere 4H-3,1-Benzoxazin-4-one, die in 2-Stellung einen gegebenenfalls substituierten Phenylrest tragen, herbizid wirksam sind (BE-A-648 259, US-A-3 970 652, US-A-3 914 121). Die bekannten Verbindungen zeichnen sich durch eine gute Verträglichkeit bei einer Reihe von Kulturen, wie Getreidearten, Reis, Mais und Kultursorghum, aus. Ihre Schwäche liegt in einem eng begrenzten Wirkungsspektrum gegen breitblättrige unerwünschte Pflanzen. Darüber hinaus sind selbst bei der Bekämpfung von Pflanzen, gegen die diese Benzoxazine wirksam sind, relativ hohe Aufwandmengen pro Flächeneinheit erforderlich.

Es wurde nun gefunden, dass 4H-3,1-Benzoxazinderivate der Formel I

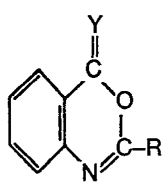

(I),

in der

Y für Sauerstoff oder Schwefel und

R für einen durch Halogenalkoxy, Halogenalkylmercapto oder Halogenalkylsulfonyl mit jeweils 1 bis 4 Kohlenstoffatomen p- oder m-substituierten Phenylrest stehen,

neben vorzüglicher Kulturpflanzenverträglichkeit eine wesentlich stärkere herbizide Wirkung zeigen als bekannte herbizide Mittel, die Benzoxazine als Wirkstoffe enthalten.

R in Formel I bedeutet beispielsweise einen Phenylrest, der folgende Substituenten in p- oder m-Stellung tragen kann:

Chlormethoxy, Fluormethoxy, Difluormethoxy, Difluorchlormethoxy, Trifluormethoxy, Trichlormethoxy, 1,1,2,2-Tetrafluoräthoxy, 1,1,2-Trifluor-2-chloräthoxy, 1,1,1-Trifluor-2-bromäthoxy, 1,1,2,3,3,3-Hexafluor-n-propyloxy, Pentafluoräthoxy, Hexafluorisopropoxy, Difluormethylmercapto, Trifluormethylmercapto, Dichlorfluormethylmercapto, Trifluormethylmercapto, Pentafluoräthylmercapto, 1,1,2,2-Tetrafluoräthylmercapto, $ClCH_2SO_2$, $F_2CHSO_2$, $CF_3SO_2$, $CF_3CF_2SO_2$.

Man erhält Benzoxazinderivate der Formel I dadurch, dass man eine Säure der Formel II

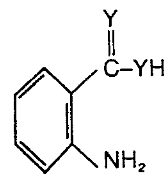

(II),

in der

Y die oben genannten Bedeutungen hat, mit mindestens dem zweifachen molaren Überschuss eines Carbonsäurehalogenids der Formel III

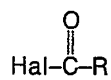

(III),

in der

R die oben genannten Bedeutungen hat und Hal für Halogen, insbesondere Fluor, Chlor oder Brom, steht, in einem aromatischen tertiären Amin als Lösungsmittel bei einer Temperatur im Bereich zwischen 10 und 60 °C umsetzt.

Zweckmässigerweise lässt man einen zweifachen Überschuss des Carbonsäurehalogenids der Formel III in eine Lösung der gegebenenfalls substituierten Anthranilsäure der Formel II in der 5- bis 25fachen molaren Menge eines aromatischen Amins, bezogen auf Anthranilsäure, bei einer Temperatur zwischen 10 und 60 °C einlaufen und rührt dann 30 Minuten bei 25 °C nach (s. J. Chem. Soc. (C) 1968, 1593). Zur Aufarbeitung kann dann Eiswasser eingerührt und vom ausgefallenen Niederschlag abgesaugt werden. Ebenso können auch das Carbonsäurehalogenid vorgelegt und die Anthranilsäure der Formel II zugegeben werden.

Geeignete aromatische tertiäre Amine sind beispielsweise Pyridin, α-, β-, γ-Picolin, Lutidin, Chinolin und Acridin.

Die Benzoxazinderivate der Formel I können auch dadurch erhalten werden, dass man eine Säure der Formel II

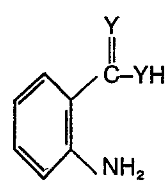

(II),

in der

Y die oben genannten Bedeutungen hat, oder ein Alkalimetall- oder Erdalkalimetallsalz dieser Anthranilsäure mit ungefähr stöchiometrischen Mengen eines Carbonsäurehalogenids der Formel III

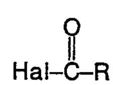

(III),

in der

R die im Anspruch 1 genannten Bedeutungen hat und Hal für Halogen steht, in einem inerten organischen Lösungsmittel oder in Wasser und gegebenenfalls in Anwesenheit eines Säureacceptors bei einer Temperatur im Bereich von 0 bis 60 °C zu einem Carbonamid der Formel IV

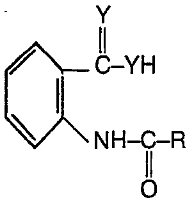

(IV),

in der

R und Y die obengenannten Bedeutungen haben, umsetzt und dieses dann in Gegenwart eines wasserentziehenden Mittels bei einer Temperatur zwischen 30 und 150 °C cyclisiert.

Geeignete inerte Lösungsmittel sind Kohlenwasserstoffe, wie Ligroin, Benzin, Toluol, Pentan, Hexan, Cyclohexan, Petroläther, Halogenkohlenwasserstoffe, wie Methylenchlorid, Chloroform, Tetrachlorkohlenstoff, 1,1- und 1,2-Dichloräthan, 1,1,1- und 1,1,2-Trichloräthan, Chlorbenzol, o-, m-, p-Dichlorbenzol, o-, m-, p-Chlortoluol, Nitrokohlenwasserstoffe, wie Nitrobenzol, Nitroäthan, o-, m-, p-Chlornitrobenzol, Nitrile, wie Acetonitril, Butyronitril, Isobutyronitril, Äther, wie Diäthyläther, Di-n-propyläther, Tetrahydrofuran, Dioxan, Ester, wie Acetessigester, Äthylacetat oder Isobutylacetat, oder Amide, wie Formamid, Methylformamid oder Dimethylformamid.

Als Säureacceptoren können alle üblichen Säurebindemittel verwendet werden. Hierzu gehören vorzugsweise Alkalihydroxide, Alkalicarbonate und tertiäre organische Basen. Als besonders geeignet seien im einzelnen genannt:
Natriumhydroxid, Natriumcarbonat, Natriumhydrogencarbonat, Triäthylamin, Pyridin, Trimethylamin, α-, β-, γ-Picolin, Lutidin, N,N-Dimethylanilin, N,N-Dimethylcyclohexylamin, Chinolin, Tri-n-propylamin und Tri-n-butylamin.
Das Säurebindemittel wird zweckmässigerweise in äquivalenten Mengen, bezogen auf Carbonsäurehalogenid der Formel III, eingesetzt.

Als wasserentziehende Mittel können symmetrische und gemischte Carbonsäureanhydride, wie Essigsäureanhydrid, Propionsäureanhydrid, Buttersäureanhydrid, Ameisensäureessigsäureanhydrid, Ameisensäurepropionsäureanhydrid oder Essigsäurepropionsäureanhydrid, ferner Dicyclohexylcarbodiimid oder Thionylchlorid verwendet werden. Die Cyclisierung wird unter Zusatz der 1- bis 10fachen molaren Menge an wasserentziehendem Mittel, bezogen auf Carbonamid der Formel IV, durchgeführt.

Die Ausgangsstoffe der Formeln II und III werden in ungefähr stöchiometrischem Verhältnis eingesetzt, d.h. in einen Unter- bzw. Überschuss von bis zu 10% Ausgangsstoff der Formel III gegenüber Ausgangsstoff der Formel II.

Zweckmässigerweise wird das Verfahren so durchgeführt, dass man über zwei Zuführungen das Carbonsäurehalogenid der Formel III und die äquivalente Menge an Säureacceptor bei einer Temperatur zwischen 0 und 60 °C zu einer ungefähr äquivalenten Menge der Anthranilsäure der Formel II bzw. ihres Salzes in einem inerten organischen Lösungsmittel bzw. in Wasser zulaufen lässt. Dann rührt man 15 Minuten bei Raumtemperatur nach. Das Reaktionsgemisch wird dann gegebenenfalls eingeengt, in der Wärme mit 5n Salzsäure angesäuert, abgekühlt und abgesaugt (J. Org. Chem. 2, 396 [1944]), wobei eine N-Acyl-2-aminobenzoesäure erhalten wird. Diese kann man in Gegenwart der 5- bis 10fachen Menge Acetanhydrid durch Rühren unter Rückfluss – gegebenenfalls unter Abdestillation der entstandenen Essigsäure – zu dem gewünschten 4H-3,1-Benzoxazin cyclisieren. Zur Aufarbeitung entfernt man hierbei überschüssiges Acetanhydrid am Rotationsverdampfer unter vermindertem Druck und kristallisiert gegebenenfalls zur Reinigung um. Anstelle der Anthranilsäure kann auch das Carbonsäurehalogenid vorgelegt werden.

Anstelle von Acetanhydrid kann man jedoch auch mit der 1- bis 4fachen Menge Dicyclohexylcarbodiimid oder Thionylchlorid bei 30 bis 150 °C cyclisieren.

Im Falle fluoralkoxy- bzw. fluoralkylmercaptosubstituierter 2-Phenyl-3,1-benzoxazin-4-one überführt man zweckmässigerweise eine fluoralkoxy- bzw. fluoralkylmercapto-substituierte Benzoesäure nach üblichen Methoden in die entsprechenden Säurechloride (Houben-Weyl, Methoden der organischen Chemie, Bd. 8, S. 463ff., 4. Auflage, Georg Thieme-Verlag, Stuttgart 1952), die dann mit gegebenenfalls substituierten Anthranilsäuren auf bekannte Weise in die entsprechenden Amide umgewandelt werden. Letztere werden dann durch Ringschluss in Gegenwart wasserabspaltender Agentien in substituierte 2-Phenyl-3,1-benzoxazin-4-one überführt.

Zur Isolierung der 4H-3,1-Benzoxazinderivate der Formel I aus der Reaktionsmischung kann man diese mit Wasser, verdünntem Alkali oder verdünnter Säure zur Abtrennung von Nebenprodukten, wie nicht umgesetzter Anthranilsäure, Säurechlorid bzw. Basenhydrochlorid, behandeln, trocknen und dann einengen. Gegebenenfalls können die Endprodukte auch durch Umkristallisation oder Chromatographie gereinigt werden.

Das folgende als Vorprodukt für 4H-3,1-Benzoxazin-4-one dienende Carbonsäurehalogenid der Formel III kann folgendermassen hergestellt werden:

3-(1′,1′,2′-Trifluor-2′-chloräthoxy)-benzoylchlorid

52,4 Gewichtsteile Chlortrifluoräthylen werden während 10 Stunden bei 45 bis 52 °C unter Rückfluss in eine Mischung von 46,5 Gewichtsteilen 3-Hydroxybenzoesäuremethylester und 9,5 Gewichtsteilen Kaliumhydroxidpulver in 50 Gewichtsteilen Aceton eingeleitet. Nach dem Einengen am Rotationsverdampfer unter vermindertem Druck wird das Reaktionsgemisch in Methylenchlorid aufgenommen, mit Natriumhydrogencarbonatlösung extrahiert, getrocknet und eingeengt, wobei 69,5 Gewichtsteile 3-(1′,1′,2′-Trifluor-2′-

chloräthoxy)-benzoesäuremethylester vom $n^{25}_D$ = 1,4710 erhalten werden.

40 Gewichtsteile 3-(1',1',2'-Trifluor-2'-chloräthoxy)-benzoesäuremethylester werden in einer Mischung von 8,4 Gewichtsteilen Kaliumhydroxid in 100 Gewichtsteilen Wasser und 5 Gewichtsteilen Tetrahydrofuran 15 Minuten bei 95 °C gerührt. Die entstandene Lösung wird mit konz. Salzsäure angesäuert, der ausgefallene Niederschlag wird abgesaugt und getrocknet; dabei werden 35 Gewichtsteile 3-(1',1',2'-Trifluor-2'-chloräthoxy)-benzoesäure vom Fp. 79 bis 85 °C isoliert.

35 Gewichtsteile 3-(1',1',2'-Trifluor-2'-chloräthoxy)-benzoesäure werden mittels 20,2 Gewichtsteilen Thionylchlorid und 0,2 Gewichtsteilen Pyridin als Katalysator in üblicher Weise in das 3-(1',1',2'-Trifluor-2'-chloräthoxy)-benzoylchlorid vom $n^{22}_D$ = 1,4900 (IR: C=O 1760, 1742 $cm^{-1}$) übergeführt; Ausbeute: 34,5 Gewichtsteile, entsprechend 92% der Theorie.

Im folgenden wird die Herstellung von 4H-3,1-Benzoxazinderivaten beispielhaft erläutert. Gewichtsteile verhalten sich zu Volumteilen wie Kilogramm zu Liter.

1. Herstellung von 2-(m-1',1',2',2'-Tetrafluoräthoxyphenyl)-3,1-benzoxazin-4-on

39,4 Gewichtsteile Thionylchlorid werden zu einer Suspension von 65 Gewichtsteilen m-(1,1,2,2-Tetrafluoräthoxy)-benzoesäure in 500 Volumteilen 1,2-Dichloräthan gegeben und dann 3 Stunden lang unter Rühren unter Rückfluss erhitzt. Nach dem Einengen unter vermindertem Druck und dem Absaugen von wenig wieder ausgefallenem Ausgangsmaterial wird das m-(1,1,2,2-Tetrafluoräthoxy)-benzoylchlorid als gelbliches Öl erhalten. Das IR-Spektrum zeigt Banden für C=O bei 1770, 1748 $cm^{-1}$, für Fluoralkoxy bei 1225, 1190, 1125 $cm^{-1}$.

25,7 Gewichtsteile m-(1,1,2,2-Tetrafluoräthoxy)-benzoylchlorid und 10,1 Gewichtsteile Triäthylamin werden unter Rühren über zwei Zuführungen innerhalb von 15 Minuten zu einer Mischung von 13,7 Gewichtsteilen Anthranilsäure in 300 Volumteilen 1,2-Dichloräthan gegeben und 12 Stunden lang bei Raumtemperatur gerührt. Das Reaktionsgemisch wird mit 0,5n Salzsäure und mit Wasser extrahiert, über Magnesiumsulfat getrocknet und dann unter vermindertem Druck eingeengt. Nach dem Anreiben in 0,5n Salzsäure, Absaugen und Waschen mit Wasser wird m-(1,1,2,2-Tetrafluoräthoxy)-benzoylanthranilsäure vom Fp. 159–163 °C erhalten.

21 Gewichtsteile des so erhaltenen Produkts werden unter Rühren während 3 Stunden mit 200 Volumteilen Acetanhydrid unter Rückfluss cyclisiert. Das Reaktionsgemisch wird unter vermindertem Druck eingeengt, in Methylenchlorid aufgenommen und über neutrales Aluminiumoxid chromatographiert. Nach dem Einengen werden 16 Gewichtsteile 2-(m-1',1',2',2'-Tetrafluoräthoxyphenyl)-3,1-benzoxazin-4-on vom Fp. 95–98 °C erhalten.

2. Herstellung von 2-(m-Difluormethoxy-phenyl)-3,1-benzoxazin-4-on

260 Gewichtsteile Chlordifluormethan werden innerhalb von 1,5 Stunden unter Rühren bei 67–70 °C in eine Mischung von 221 Gewichtsteilen m-Kresol, 412 Gewichtsteilen Natriumhydroxid, 600 Volumteilen 1,4-Dioxan und 500 Volumteilen Wasser eingeleitet. Nach 45 Minuten Rühren bei 68 °C wird das Reaktionsgemisch abgekühlt, mit 1000 Volumteilen Wasser verdünnt und viermal mit 200 Volumteilen Äther extrahiert. Nach dem Trocknen, Einengen unter vermindertem Druck und Destillieren werden 172 Gewichtsteile m-Tolyldifluormethyläther vom Sdp. 64–67 °C/24,7 mbar erhalten.

Eine Mischung von 47,4 Gewichtsteilen m-Tolyldifluormethyläther, 77 Gewichtsteilen Magnesiumsulfat, 134,3 Gewichtsteilen Kaliumpermanganat und 1900 Volumteilen Wasser werden 3 Stunden lang bei 50–60 °C und 2 Stunden lang bei 90 °C gerührt. Nach dem Vernichten überschüssigen Permanganats mit Äthanol wird die Lösung noch heiss abgesaugt und dann das Filtrat angesäuert. Der ausgefallene Niederschlag wird in Methylenchlorid aufgenommen und getrocknet, wobei nach dem Einengen unter vermindertem Druck die 3-Difluormethoxybenzoesäure vom Fp. 85–87 °C erhalten wird.

Die so erhaltene 3-Difluormethoxy-benzoesäure lässt sich mittels Thionylchlorid auf übliche Weise in das 3-Difluormethoxybenzoylchlorid vom $n^{25}_D$ = 1,5083 überführen.

25 Gewichtsteile 3-Difluormethoxybenzoylchlorid und 12,2 Gewichtsteile Triäthylamin werden über 2 Zuführungen unter Rühren bei 25 bis 30 °C zu einer Mischung von 16,6 Gewichtsteilen Anthranilsäure in 360 Gewichtsteilen 1,2-Dichloräthan innerhalb 15 Minuten gegeben. Nach 2 Stunden Rühren bei 25 °C wird das Reaktionsgemisch mit 0,5n Salzsäure und mit Wasser extrahiert. Anschliessend wird die organische Phase viermal mit je 100 Teilen 0,5n Natronlauge extrahiert und letztere in verd. Salzsäure eingerührt. Nach dem Absaugen und Trocknen werden 30,4 Gewichtsteile entsprechend 85% d.Th., N-(3-Difluormethoxybenzoyl)-anthranilsäure vom Fp. 186 bis 191 °C erhalten.

8,33 Gewichtsteile Thionylchlorid werden unter Rühren bei 25 °C in eine Mischung von 18 Gewichtsteilen N-(3-Difluormethoxybenzoyl)-anthranilsäure in 250 Gewichtsteilen 1,2-Dichloräthan eingeführt; dann wird 4 Stunden unter Rückfluss gerührt. Nach dem Abkühlen wird das Reaktionsgemisch mit 100 Volumteilen Eiswasser und 100 Volumteilen 0,5n Natronlauge extrahiert, getrocknet und über neutralem Aluminiumoxid chromatographiert. Dabei werden 12 Gewichtsteile, entsprechend 71% der Theorie, 2-(m-Difluormethoxyphenyl)-3,1-benzoxazin-4-on vom Fp. 84–87 °C erhalten.

Nach entsprechenden Verfahren können folgende 4H-3,1-Benzoxazinderivate der Formel I hergestellt werden.

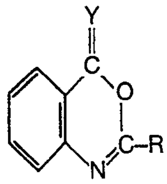

| R | Y | Fp. [°C] |
|---|---|---|
| 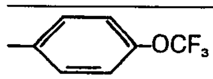 | O | 87– 90 |
| 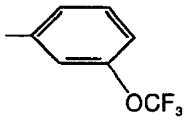 | O | 94– 95 |
| 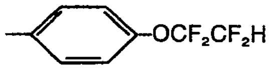 | O | 98–102 |
| 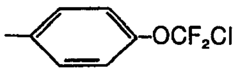 | O | |
| 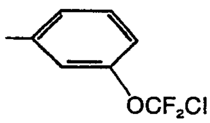 | O | 82– 86 |
| 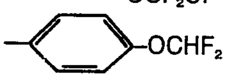 | O | |
| 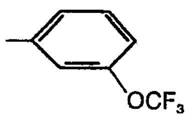 | S | |
| 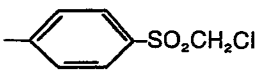 | O | |
| 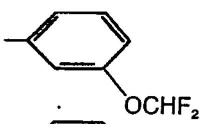 | O | |
| 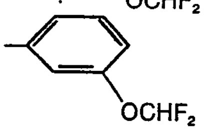 | S | |
| 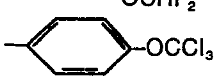 | O | 145–149 |
| 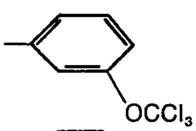 | O | 107–110 |
| 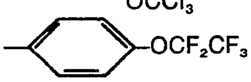 | O | |
| 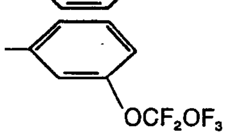 | O | 108–112 |
| 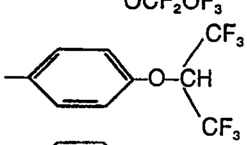 | S | |
| 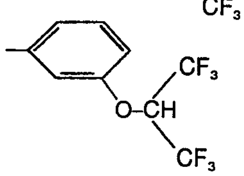 | O | |

| R | Y | Fp. [°C] |
|---|---|---|
| 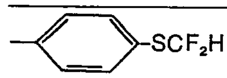 | O | |
| 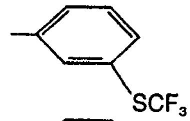 | O | 87– 90 |
| 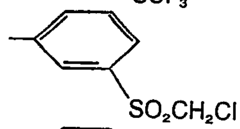 | O | |
| 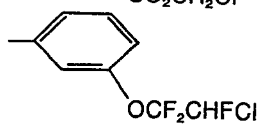 | O | 105–108 |
| 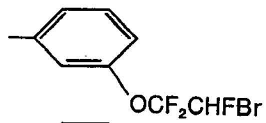 | O | 86– 89 |
| 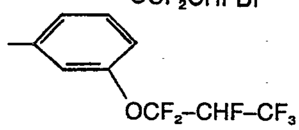 | O | 88– 91 |
| 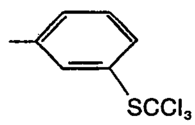 | O | 130–134 |
| 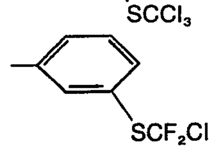 | O | |
| 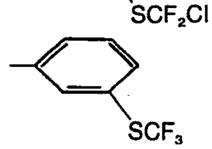 | S | |
| 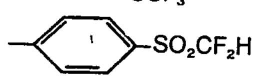 | O | |
| 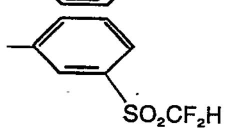 | O | |
| 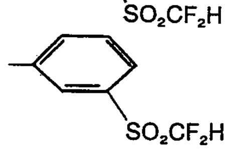 | S | |
| 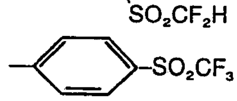 | O | |
| 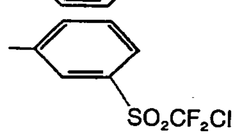 | O | |
| 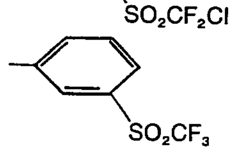 | S | |
| 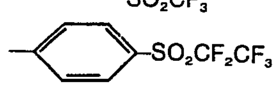 | O | |
| 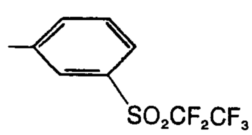 | O | |

Die Wirkstoffe können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt wie Kerosin oder Dieselöl, ferner Kohlenteeröle, sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind Mineralerden wie Silicagel, Kieselsäuren, Kiselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehl, Cellulosepulver und andere feste Trägerstoffe.

Die erfindungsgemässen Mittel enthalten als Formulierungen zwischen 0,1 und 95 Gewichtsprozent, vorzugsweise zwischen 0,5 und 90 Gewichtsprozent, Wirkstoff.

Beispiele für Formulierungen sind:

I. 20 Gewichtsteile der Verbindung gemäss Beispiel 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

II. 20 Gewichtsteile der Verbindung gemäss Beispiel 2 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Äthylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

III. 20 Teile der Verbindung gemäss Beispiel 1 werden mit 2 Teilen Calciumsalz der Dodecylbenzolsulfonsäure, 8 Teilen Fettalkohol-polyglykoläther, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Der Einfluss der erfindungsgemässen Mittel auf das Wachstum von unerwünschten Pflanzen wird durch Gewächshausversuche gezeigt:

Als Kulturgefässe dienten Plastikblumentöpfe mit 300 $cm^3$ Inhalt und lehmigem Sand mit etwa 1,5% Humus als Substrat. Die Samen der Testpflanzen entsprechend Tabelle 1 wurden nach Arten getrennt flach eingesät oder es wurden transplantierte vorgekeimte Jungpflanzen oder Stecklinge transplantiert. Generell wurden sie zum Zwecke der Nachauflaufbehandlung bis zu einer

Höhe von 3 bis 10 cm gezogen und danach behandelt. Die Wirkstoffe wurden auf die Sprossteile der Pflanzen und die nicht völlig von Pflanzen abgedeckte Bodenoberfläche, in Wasser als Verteilungsmittel suspendiert oder emulgiert, mittels fein verteilender Düsen gespritzt. Die Versuchstöpfe wurden dann in verschiedenen Temperaturbereichen der Gewächshausanlagen aufgestellt, wobei für wärmeliebende Arten 20 bis 30 °C und für solche gemässigter Klimate 10 bis 20 °C bevorzugt wurden. Die Versuchsperiode erstreckte sich über 2 bis 4 Wochen. Während dieser Zeit wurden die Pflanzen gepflegt, und ihre Reaktion auf die einzelnen Wirkstoffe wurde ausgewertet. Die Auswertung der Versuche wurde nach einer Skala von 0 bis 100 vorgenommen. Dabei bedeutet 0 keine Schädigung und 100 völliges Absterben zumindest der oberirdischen Sprossteile.

Die in den Versuchen getesteten Pflanzenarten sind in Tabelle 1 aufgeführt.

Die Versuchsergebnisse in den Tabellen zeigen, dass Herbizide, die 4H-3,1-Benzoxazinderivate der Formel I enthalten, sich im Vergleich zu herbiziden Mitteln, die bekannte herbizide Benzoxazine enthalten, durch bessere herbizide Wirkung mit guter Selektivität bei einer Reihe von Kulturpflanzen auszeichnen. Der Schwerpunkt der Anwendung liegt in der Nachauflaufbehandlung der unerwünschten Pflanzen, sowohl auf Kulturflächen als auch auf unbebautem Land.

Sind die Kulturpflanzen gegenüber den Wirkstoffen weniger tolerant, so können auch Ausbringungstechniken angewandt werden, bei welchen die herbiziden Mittel mit Hilfe der Spritzgeräte so geleitet werden, dass die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post directed, lay-by).

Tabelle 1 – Liste der Pflanzennamen

| Botanischer Name | Abkürzung in Tabellen | Deutscher Name | Englischer Name |
|---|---|---|---|
| Acanthospermum hispidum | Acanthosp. hisp. | | bristly starbur |
| Avena sativa | | Hafer | oats |
| Beta vulgaris | Beta vulg. | Zuckerrübe | sugarbeets |
| Centaurea spp. | | Flockenblumenarten | knapweed |
| Chenopodium album | Chenopod. album | Weisser Gänsefuss | lambsquarters (goosefoot) |
| Datura stramomium | Datura stram. | Gemeiner Stechapfel | Jimsonweed |
| Desmodium tortuosum | Desmod. tort. | | Florida beggarweed |
| Euphorbia geniculata | Euphorb. genic | südamerik. Wolfsmilchart | wild poinsettia |
| Glycine max | | Sojabohnen | soybeans |
| Galeopsis spp. | | Hohlzahnarten | hemp-nettle |
| Gossypium hirsutum | Gossyp. hirs. | Baumwolle | cotton |
| Hordeum vulgare | | Gerste | barley |
| Matricarla spp. | Matric. spp. | Kamillearten | chamomille |
| Malva neglecta | | | common mallow |
| Mercurialis annua | Mercurial. annua | Einjähriges Bingelkraut | annual mercury |
| Oryza sativa | | Reis | rice |
| Sesbania exaltata | | Turibaum | hemp sesbania (coffeeweed) |
| Solanum nigrum | Solan. nigr. | Schwarzer Nachtschatten | black nightshade |
| Sorghum bicolor | | Mohrenhirse (Kulturhirse) | sorghum |
| Triticum aestivum | | Weizen | wheat |
| Xanthium pensylvanicum | Xanthium pens. | Spitzklette | common cocklebur |
| Zea mays | | Mais | Indian corn |

Tabelle 2 – Selektive herbizide Wirkung neuer Verbindungen bei Nachaufnahmen im Gewächshaus

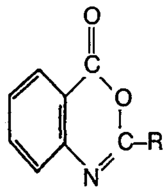

| R | Kulturpflanzen-Schädigung in % bei 1,0 kg a.S./ha Aufwandmenge Hordeum vulgare | Oryza sativa | Sorghum bicolor | Triticum aestivum | Zea mays | Index der herbiziden Wirkung bei 0,5 kg/ha a.S. |
|---|---|---|---|---|---|---|
| 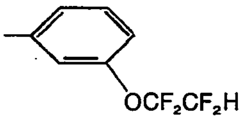 | 0 | 2 | 0 | 10 | 17 | 87 |
| 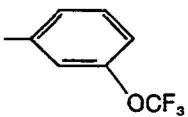 | 0 | 0 | 0 | 0 | 9 | 90 |
| 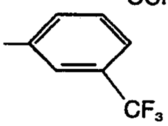 (bekannt)** | 0 | 5 | 30 | 23 | 18 | 58 |

0 = keine Schädigung, 100 = Pflanzen abgestorben

* errechnet aus Durchschnittswerten der Pflanzen: Chenopodium album, Cyperus spp., Chrysanthemum segetum, Datura stramonium, Matricaria spp., Mercurialis annua, Sesbania exaltata und Solanum nigrum

** US-A-3 941 121

Tabelle 3 – Selektive Bekämpfung wichtiger breitblättriger Unkräuter in Soja bei Nachauflaufanwendung im Gewächshaus

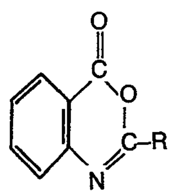

| R | Aufwandmenge [kg a.S./ha] | Schädigung in % an den Testpflanzen Glycine max | Cheno-pod. album | Datura stram. | Euphorbia geniculata | Solanum nigrum | Xanthium pens. |
|---|---|---|---|---|---|---|---|
| 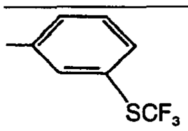 | 0,5 | 12 | 99 | 100 | 92 | 100 | 100 |
| 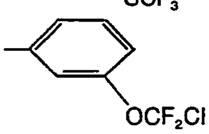 | 0,5 | 8 | 70 | 100 | 99 | 100 | 100 |
| 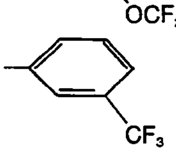 (bekannt)* | 0,5 | 21 | 89 | 87 | 17 | 90 | |

0 = keine Schädigung

100 = Pflanzen abgestorben

* US-A-3 914 121

Tabelle 4 – Selektive Bekämpfung von Galeopsis spp. bei Anwendung im Nachauflaufverfahren im Gewächshaus

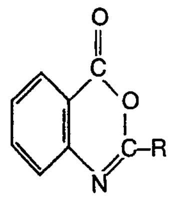

| R | Aufwandmenge [kg a. S./ha] | Schädigung in % an den Testpflanzen Hordeum vulgare | Triticum aestivum | Galeopsis spp. |
|---|---|---|---|---|
| 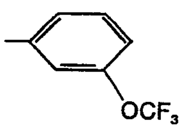 | 0,5 | 0 | 10 | 90 |
| | 1,0 | 0 | 10 | 94 |
| 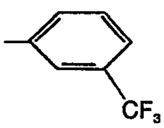 (bekannt)* | 0,5 | 0 | 20 | 30 |
| | 1,0 | 0 | 23 | 40 |

* US-A-3 914 121

Tabelle 5 – Selektive herbizide Wirkung von 4H-3,1-Benzoxazinderivaten bei Nachauflaufanwendung im Gewächshaus

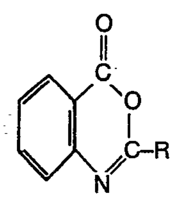

| R | Aufwandmenge [kg a. S./ha] | Schädigung (in %) an den Testpflanzen Avena sativa | Centaurea spp. |
|---|---|---|---|
| 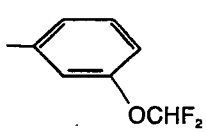 | 3,0 | 20 | 70 |
| 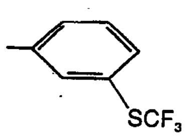 | 3,0 | 40 | 100 |
| 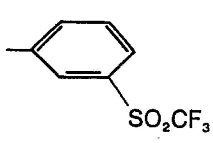 | 3,0 | 80 | 90 |

Tabelle 6 – Selektive Bekämpfung unerwünschter Pflanzen bei Nachauflaufanwendung im Gewächshaus

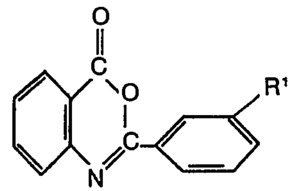

| $R^1$ | Aufwandmenge [kg a.S./ha] | Schädigung in % an den Testpflanzen Zea mays | Chenop. album | Desmod. tort. | Euphorb. genic. | Matric. spp. | Mercurial. annua | Malva neglecta | Solanum nigrum |
|---|---|---|---|---|---|---|---|---|---|
| $-OCF_2CF_3$ | 0,5 | 0 | 100 | 100 | 100 | 100 | 100 | 100 | 100 |
| $-OCF_2CFHCl$ | 0,5 | 0 | 99 | 100 | 98 | – | 90 | | 95 |

0 = keine Schädigung
100 = Pflanzen gestorben

Tabelle 7 – Bekämpfung unerwünschter Pflanzen in Baumwolle bei Nachauflaufanwendung im Gewächshaus

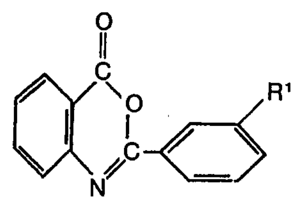

| $R^1$ | Aufwandmenge [kg a.S./ha] | Schädigung in % an den Testpflanzen Gossyp. hirs. | Acanthosp. hisp. | Chenop. alb. | Datura stram. | Euphorb. gen. | Solan. nigr. | Xanthium pens. | Sesbania exalt. |
|---|---|---|---|---|---|---|---|---|---|
| $SO_2CF_3$ | 1,0 | 0 | 100 | 87 | 100 | 79 | 93 | 100 | 73 |
| $CF_3$ | 1,0 | 43 | 100 | 97 | 80 | 26 | 99 | 99 | 67 |

0 = keine Schädigung
100 = Pflanzen abgestorben

Tabelle 8 – Selektive Bekämpfung von Unkräutern in Zuckerrüben bei Nachauflaufanwendung im Gewächshaus

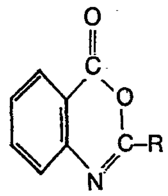

| R | Aufwandmenge [kg a.S./ha] | Schädigung in % an den Testpflanzen Beta vulg. | Chenopodium album | Solanum nigrum |
|---|---|---|---|---|
| 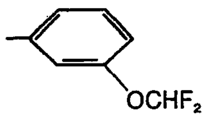 | 2,0 | 10 | 85 | 100 |

0 = keine Schädigung
100 = Pflanzen abgestorben

In Anbetracht der guten Verträglichkeit und der Vielseitigkeit der Applikationsmethoden können die erfindungsgemässen Herbizide ausser bei den in Tabelle 1 aufgeführten Nutzpflanzen noch in einer weiteren grossen Zahl von Kulturpflanzen zur Beseitigung unerwünschten Pflanzenwuchses eingesetzt werden. Die Aufwandmengen können dabei zwischen 0,1 und 15 kg/ha und mehr schwanken.

In Betracht kommen beispielsweise die folgenden Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape |
| Brassica napus var. napobrassica | Kohlrübe | |
| Brassica napus var. rapa | Weisse Rübe | turnips |
| Brassica rapa var. silvestris | Rübsen | |
| Camellia sinensis | Teestrauch | tea plants |
| Carthamus tinctorius | Saflor – Färberdistel | safflower |
| Carya illinoinensis | Pekannussbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |

| Botanischer Name | Deutscher Name | Englischer Name |
|---|---|---|
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactuca sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot exculenta | Maniok | cassava |
| Medicago sativa | Luzerne | alfalfa (lucerne) |
| Mentha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolbenhirse | |
| Petroselinum crispum spp. tuberosum | Wurzelpetersilie | parsley |
| Picea abies | Rotfichte | Norway spruce |
| Abies alba | Weisstanne | fir |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes silvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium corymbosum | Kulturheidelbeere | blueberry |
| Vaccinium vitis-idaea | Preisselbeere | cranberry |
| Vicia faba | Pferdebohnen | tick beans |
| Vigna sinensis (v. unguiculata) | Kuhbohne | cow peas |
| Vitis vinifera | Weinrebe | grapes |
| Zea mays | Mais | Indian corn, sweet corn, maize |

Zur Verbreiterung des Wirkungsspektrums und gegebenenfalls zur Erzielung synergistischer Effekte können die 4H-3,1-Benzoxazinderivate der Formel I sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, Benzothiadiazinone, 2,6-Dinitroaniline, N-Phenylcarbamate, Thiolcarbamate, Halogencarbonsäuren, Triazine, Amide, Harnstoffe, Diphenyläther, Triazinone, Uracile, Benzofuranderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

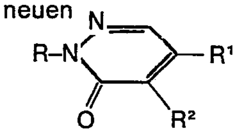

| R | $R^1$ | $R^2$ |
|---|---|---|
| 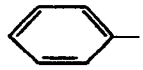 | $NH_2$ | Cl |
| 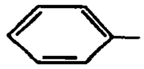 | $NH_2$ | Br |
| 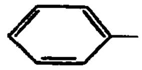 | $OCH_3$ | $OCH_3$ |
| 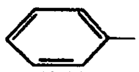 | $N(CH_3)_2$ | Cl |
| 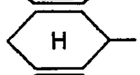 | $OCH_3$ | $OCH_3$ |
| 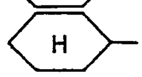 | $NH_2$ | Cl |
| 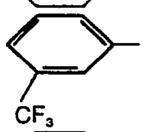 | $N(CH_3)_2$ | Cl |
| 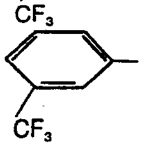 | $NHCH_3$ | Cl |
| 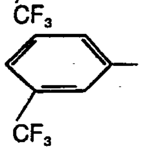 | $OCH_3$ | Cl |
| 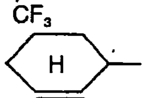 | $NH_2$ | Br |
| 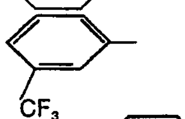 | $OCH_3$ | $OCH_3$ |
| 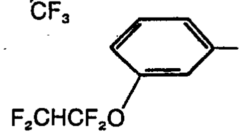 | $NHCH_3$ | Cl |

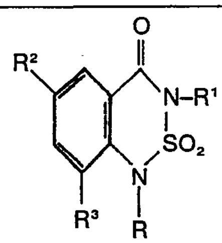

| R | $R^1$ | $R^2$ | $R^3$ |
|---|---|---|---|
| H | $i\text{–}C_3H_7$ | H | H (Salze) |
| H | $i\text{–}C_3H_7$ | H | $CH_3$ (Salze) |
| H | $i\text{–}C_3H_7$ | H | Cl (Salze) |
| $CH_2\text{–}OCH_3$ | $i\text{–}C_3H_7$ | H | H |
| H | $i\text{–}C_3H_7$ | H | F (Salze) |
| $CH_2\text{–}OCH_3$ | $i\text{–}C_3H_7$ | H | Cl |
| $CH_2\text{–}OCH_3$ | $i\text{–}C_3H_7$ | H | F |
| CN | $i\text{–}C_3H_7$ | H | Cl |

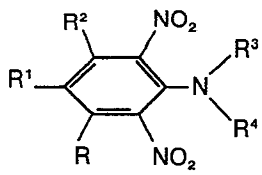

| R | R¹ | R² | R³ | R⁴ |
|---|---|---|---|---|
| H | $H_3CSO_2$ | H | $n-C_3H_7$ | $n-C_3H_7$ |
| H | $F_3C$ | H | $C_2H_5$ | $C_4H_9$ |
| H | $F_3C$ | H | $n-C_3H_7$ | $n-C_3H_7$ |
| H | $F_3C$ | H | $-CH_2-CH_2Cl$ | $n-C_3H_7$ |
| H | $tert-C_4H_9$ | H | $sec-C_4H_9$ | $sec-C_4H_9$ |
| H | $SO_2NH_2$ | H | $n-C_3H_7$ | $n-C_3H_7$ |
| H | $F_3C$ | H | $n-C_3H_7$ | $-CH_2-$ 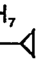 |
| $H_3C$ | $H_3C$ | H | H | $sec-C_4H_9$ |
| $H_3C$ | $H_3C$ | H | H | $-CH(C_2H_5)_2$ |
| H | $F_3C$ | $NH_2$ | $n-C_3H_7$ | $n-C_3H_7$ |
| H | $H_3C$ | H | $n-C_3H_7$ | $n-C_3H_7$ |
| H | $i-C_3H_7$ | H | $n-C_3H_7$ | $n-C_3H_7$ |

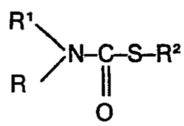

| R | R¹ | R² |
|---|---|---|
| 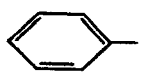 | H | $i-C_3H_7$ |
| $CH_3$ | H | $-CH_2-$ 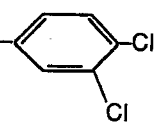 |
| 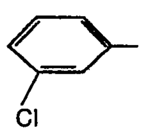 | H | 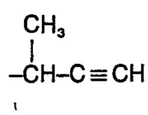 |
| 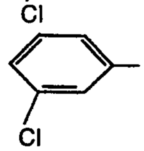 | H | $-CH_2-C\equiv C-CH_2Cl$ |
| 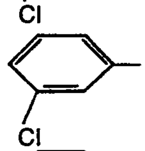 | H | $i-C_3H_7$ |
| 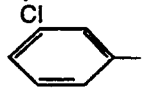 | H | 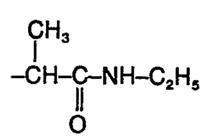 |
| 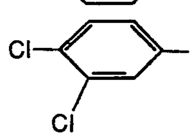 | H | $CH_3$ |
| 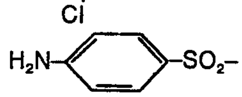 | H | $CH_3$ |
| $CH_3$ | H | 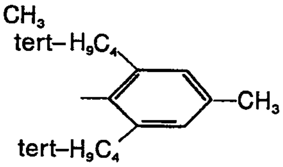 |
| 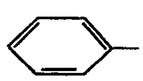 | H | 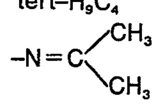 |

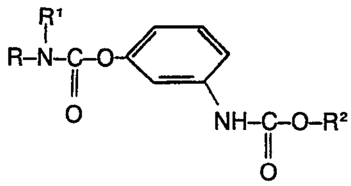

| R | R¹ | R² |
|---|---|---|
| 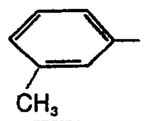 | H | $CH_3$ |
| 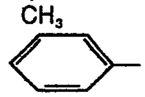 | H | $C_2H_5$ |
| 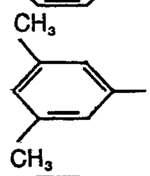 | H | $C_2H_5$ |
| 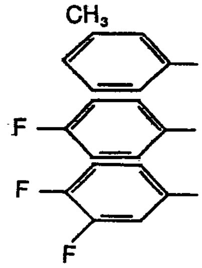 | $CH_3$ | $CH_3$ |
| | H | $CH_3$ |
| | H | $C_2H_5$ |
| 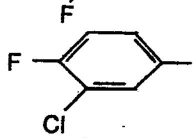 | H | $C_2H_5$ |
| 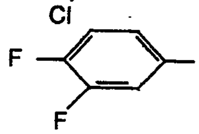 | H | $CH_3$ |
| 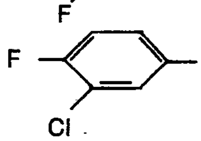 | H | $CH_3$ |

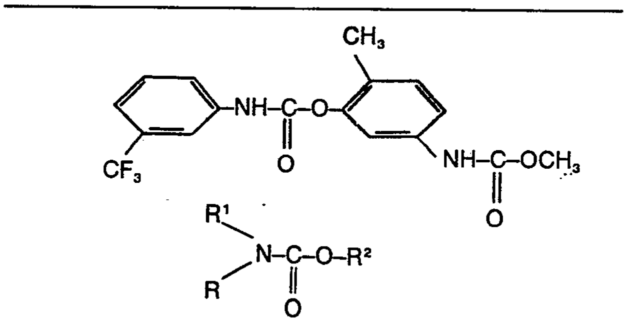

| R | R¹ | R² |
|---|---|---|
| $i-C_3H_7$ | $i-C_3H_7$ | $-CH_2-CCl=CCl_2$ |
| $i-C_3H_7$ | $i-C_3H_7$ | $-CH_2-CCl=CHCl$ |
| $n-C_3H_7$ | $n-C_3H_7$ | $C_2H_5$ |
| 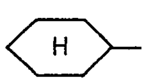 | $C_2H_5$ | $C_2H_5$ |
| $sec-C_4H_9$ | $sec-C_4H_9$ | $C_2H_5$ |
| $n-C_3H_7$ | $n-C_3H_7$ | $n-C_3H_7$ |

| R | $R^1$ | $R^2$ |
|---|---|---|
| $C_2H_5$ | $C_2H_5$ | 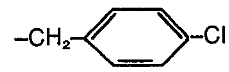 |
| sec–$C_4H_9$ | sec–$C_4H_9$ | 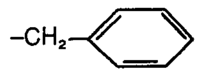 |
| 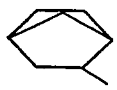 | $C_2H_5$ | $C_2H_5$ |
| i–$C_3H_7$ | i–$C_3H_7$ | 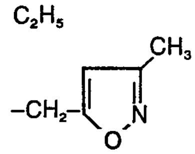 |
| i–$C_3H_7$ | i–$C_3H_7$ | 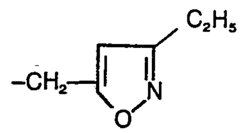 |

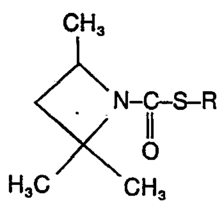

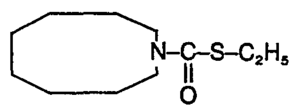

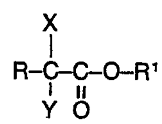

R = $-CH_2-CCl=CHCl$, $-CH_2-CCl=CCl_2$

| R | X | Y | $R^1$ |
|---|---|---|---|
| $CH_3$ | Cl | Cl | Na |
| 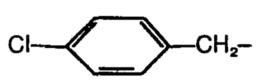 | Cl | H | $CH_3$ |
| 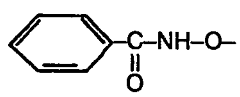 | H | H | H (Salze) |
| Cl | Cl | Cl | Na |
| 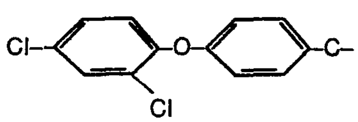 | H | $CH_3$ | $CH_3$ |
| 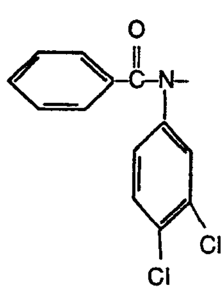 | H | $CH_3$ | $C_2H_5$ |
| $C_2H_5$ | Cl | Cl | Na |

| R | X | Y | $R^1$ |
|---|---|---|---|
| 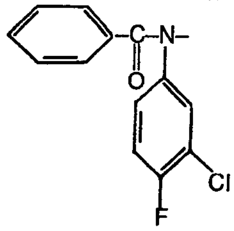 | H | $CH_3$ | $i-C_3H_7$ |
| 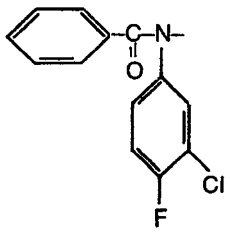 | H | $CH_3$ | $CH_3$ |
| 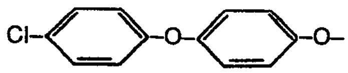 | H | $CH_3$ | $-CH_2-CH(CH_3)_2$ |
| 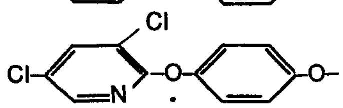 | H | $CH_3$ | Na |
| 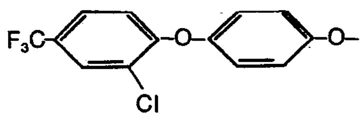 | H | $CH_3$ | Na |
| 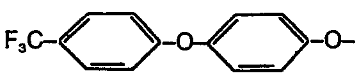 | H | $CH_3$ | $CH_3$ |

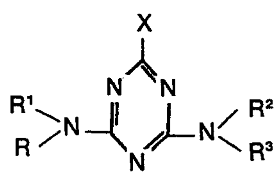

| R | $R^1$ | X | $R^2$ | $R^3$ |
|---|---|---|---|---|
| H | $tert-C_4H_9$ | $SCH_3$ | H | $C_2H_5$ |
| H | $C_2H_5$ | $SCH_3$ | H | $C_2H_5$ |
| H | $i-C_3H_7$ | $SCH_3$ | H | $C_2H_5$ |
| H | $CH_3$ | $SCH_3$ | H | $i-C_3H_7$ |
| H | $i-C_3H_7$ | Cl | H | $C_{2h}5$ |
| H | $i-C_3H_7$ | Cl | H | 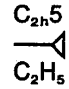 |
| H | $C_2H_5$ | Cl | H | $C_2H_5$ |
| H | $C_2H_5$ | Cl | H | $CH_3$<br>\|<br>$-C-CN$<br>\|<br>$CH_3$ |
| H | $i-C_3H_7$ | Cl | H | $i-C_3H_7$ |
| H | $i-C_3H_7$ | $OCH_3$ | H | $i-C_3H_7$ |
| H | $CH_3$<br>\|<br>$NC-C-$<br>\|<br>$CH_3$ | Cl | H | 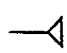 |
| H | $C_2H_5$ | Cl | H | $CH_3$<br>\|<br>$-CH-CH_2-OCH_3$ |
| H | $C_2H_5$ | Cl | H | $CH_3$<br>\|<br>$-CH-C \equiv CH$ |

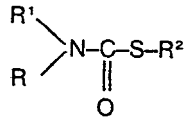

| R | $R^1$ | $R^2$ |
|---|---|---|
| $CH_3$ | $CH_3$ | $CH(C_6H_5)_2$ |
| 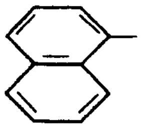 | H | 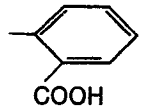 |
| 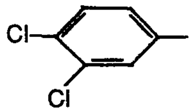 | H | 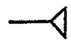 |
| 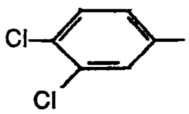 | H | $C_2H_5$ |
| 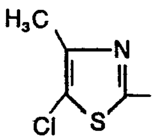 | H | $C_2H_5$ |
| 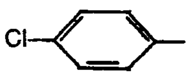 | H | 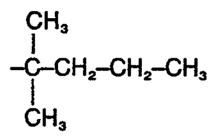 |
| 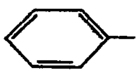 | 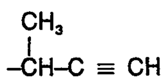 | $CH_2Cl$ |
| 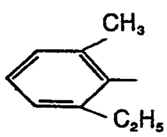 | 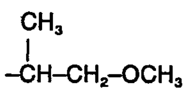 | $CH_2Cl$ |
| 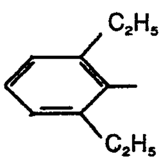 | $-CH_2-OCH_3$ | $CH_2Cl$ |
| 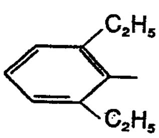 | 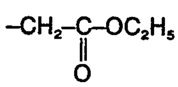 | $CH_2Cl$ |
| 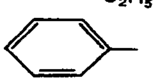 | $i-C_3H_7$ | $CH_2Cl$ |
| 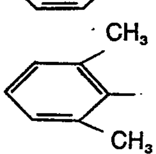 | $-CH_2-O-CH_2-CH(CH_3)_2$ | $CH_2Cl$ |
| 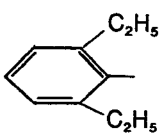 | $-CH_2-O-n-C_4H_9$ | $CH_2Cl$ |
| 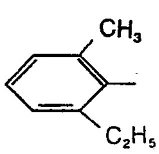 | $-CH_2-O-C_2H_5$ | $CH_2Cl$ |

| R | $R^1$ | $R^2$ |
|---|---|---|
| 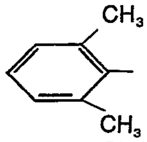 | 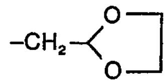 | $CH_2Cl$ |
| 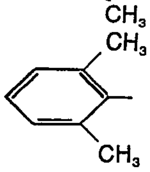 | $-CH_2-CH_2-OCH_3$ | $CH_2Cl$ |
| 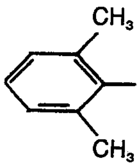 | 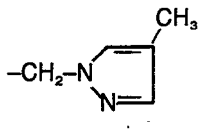 | $CH_2Cl$ |
| 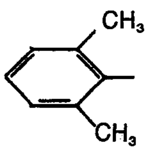 | 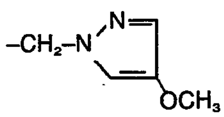 | $CH_2Cl$ |
| 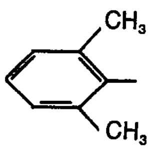 | 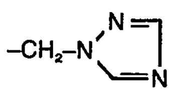 | $CH_2Cl$ |
| 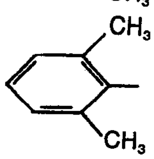 | 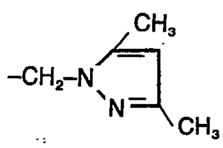 | $CH_2Cl$ |
| $CH_3$ | $CH_3$ | 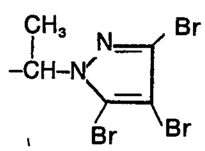 |
| $C_2H_5$ | $C_2H_5$ | 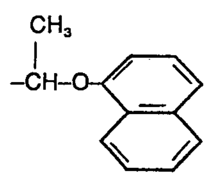 |
| $CH_2=CH-CH_2-$ | $CH_2=CH-CH_2-$ | $CH_2Cl$ |
| 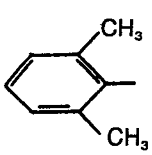 | 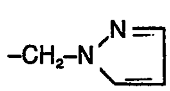 | $CH_2Cl$ |
| 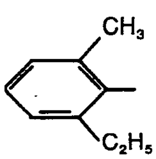 | 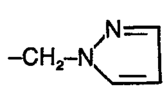 | $CH_2Cl$ |
| 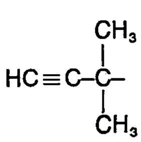 | H | 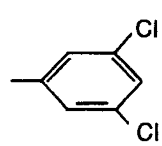 |

| R | $R^1$ | $R^2$ |
|---|---|---|
| 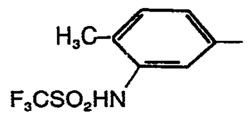 | H | $CH_3$ |
| 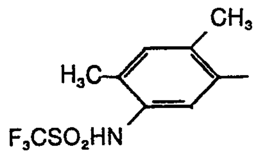 | H | $CH_3$ |

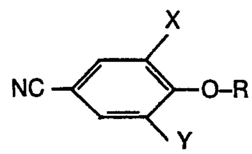

| X | Y | R |
|---|---|---|
| Br | Br | H (Salze) |
| J | J | H (Salze) |
| Br | Br | 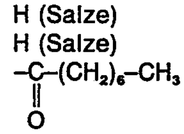 |
| 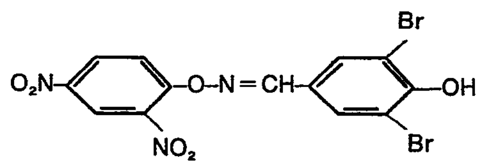 | | Salze, Ester |
| 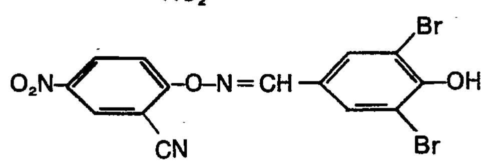 | | Salze, Ester |

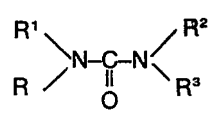

| R | $R^1$ | $R^2$ | $R^3$ |
|---|---|---|---|
| 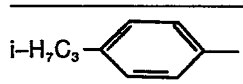 | H | $CH_3$ | $CH_3$ |
| 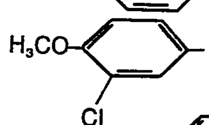 | H | $CH_3$ | $CH_3$ |
| 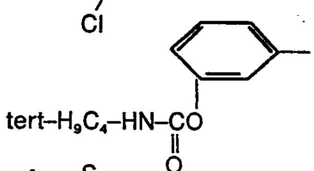 | $CH_3$ | $CH_3$ | $CH_3$ |
| 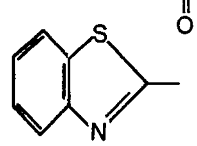 | H | $CH_3$ | H |
| 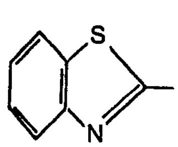 | $CH_3$ | $CH_3$ | H |

| R | R¹ | R² | R³ |
|---|---|---|---|
| 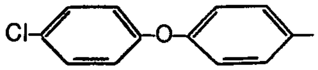 | H | $CH_3$ | $CH_3$ |
| 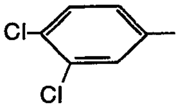 | H | $CH_3$ | $CH_3$ |
| 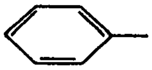 | H | 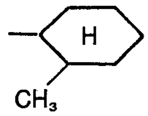 | H |
| 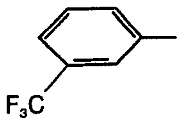 | H | $CH_3$ | $CH_3$ |
| 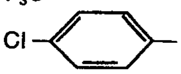 | H | $CH_3$ | 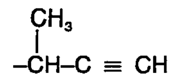 |
| 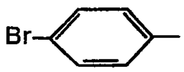 | H | $CH_3$ | $OCH_3$ |
| 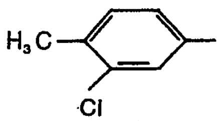 | H | $CH_3$ | $CH_3$ |
| 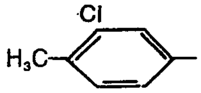 | H | 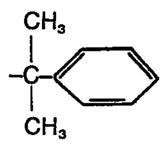 | H |
| 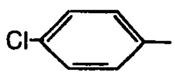 | H | $CH_3$ | $OCH_3$ |
| 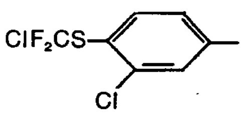 | H | $CH_3$ | $CH_3$ |
| 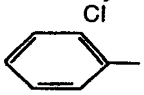 | H | $CH_3$ | $CH_3$ |
| 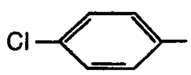 | H | $CH_3$ | $CH_3$ |
| 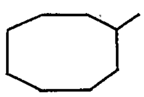 | H | $CH_3$ | $CH_3$ |
| 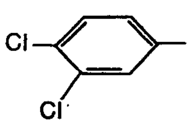 | H | $CH_3$ | $OCH_3$ |
| 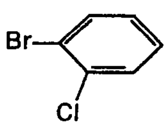 | H | $CH_3$ | $OCH_3$ |
| 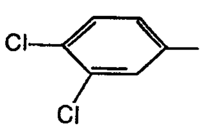 | H | $CH_3$ | H |
| 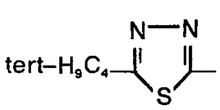 | $CH_3$ | $CH_3$ | H |
| 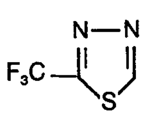 | $CH_3$ | $CH_3$ | H |

| R | $R^1$ | $R^2$ | $R^3$ |
|---|---|---|---|
| 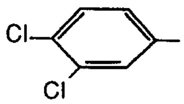 | H | $C_2H_5$ | $C_2H_5$ |
| 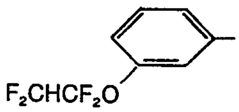 | H | $CH_3$ | $CH_3$ |
| 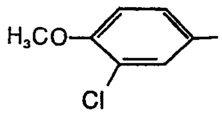 | H | $CH_3$ | $OCH_3$ |
| 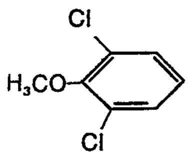 | H | $CH_3$ | $CH_3$ |

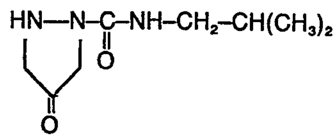

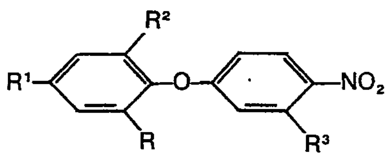

| R | $R^1$ | $R^2$ | $R^3$ |
|---|---|---|---|
| Cl | Cl | Cl | H |
| F | Cl | Cl | H |
| $NO_2$ | $CF_3$ | H | H |
| Cl | $CF_3$ | H | COOH (Salze) |
| Cl | Cl | H | H |
| Cl | Cl | H | $OCH_3$ |
| Cl | Cl | H | 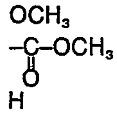 |
| H | $CF_3$ | Cl | H |
| H | $CF_3$ | Cl | $OC_2H_5$ |

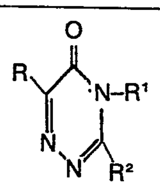

| R | $R^1$ | $R^2$ |
|---|---|---|
| tert–$C_4H_9$ | $NH_2$ | $SCH_3$ |
| tert–$C_4H_9$ | $-N=CH-CH(CH_3)_2$ | $SCH_3$ |
| 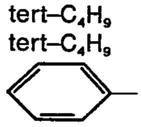 | $NH_2$ | $CH_3$ |

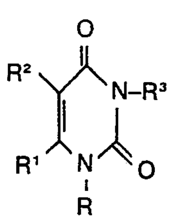

| R | $R^1$ | $R^2$ | $R^3$ |
|---|---|---|---|
| H | $CH_3$ | Br | 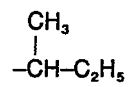 |
| H | $CH_3$ | Br | i–$C_3H_7$ |
| H | $CH_3$ | Cl | tert–$C_4H_9$ |
| H | $CH_3$ | Cl | 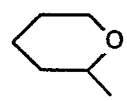 |

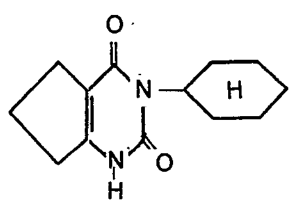

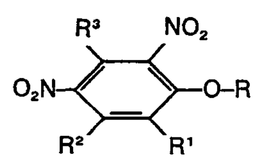

| R | $R^1$ | $R^2$ | $R^3$ |
|---|---|---|---|
| 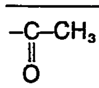 | sec–$C_4H_9$ | H | H |
| H | $CH_3$ | H | H (Salze, Ester) |
| H | sec–$C_4H_9$ | H | H (Salze, Ester) |
| 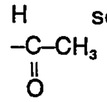 | tert–$C_4H_9$ | H | H |
| 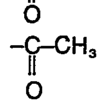 | tert–$C_4H_9$ | H | $CH_3$ |
| H | i–$C_3H_7$ | $CH_3$ | H (Salze, Ester) |
| H | tert–$C_4H_9$ | H | H (Salze) |

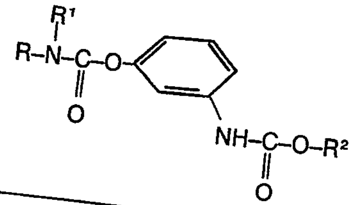

| R | $R^1$ | $R^2$ |
|---|---|---|
| 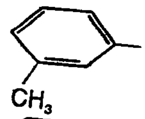 | H | $CH_3$ |
| 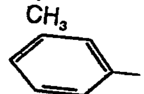 | H | $C_2H_5$ |
| 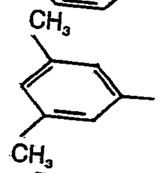 | H | $C_2H_5$ |
| 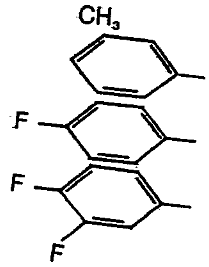 | $CH_3$ | $CH_3$ |
| | H | $CH_3$ |
| | H | $C_2H_5$ |
| 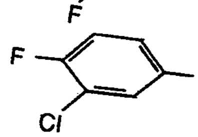 | H | $C_2H_5$ |
| 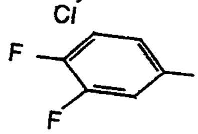 | H | $CH_3$ |
| 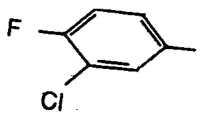 | H | $CH_3$ |

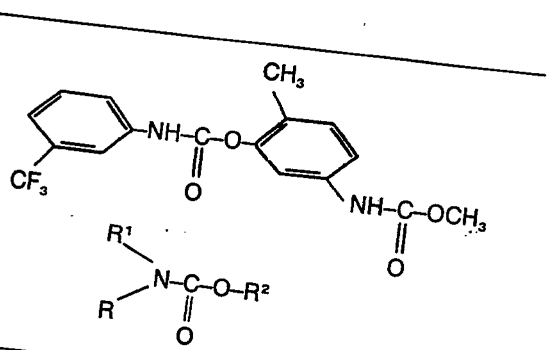

| R | $R^1$ | $R^2$ |
|---|---|---|
| $i-C_3H_7$ | $i-C_3H_7$ | $-CH_2-CCl=CCl_2$ |
| $i-C_3H_7$ | $i-C_3H_7$ | $-CH_2-CCl=CHCl$ |
| $n-C_3H_7$ | $n-C_3H_7$ | $C_2H_5$ |
| 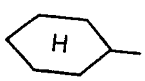 | $C_2H_5$ | $C_2H_5$ |
| $sec-C_4H_9$ | $sec-C_4H_9$ | $C_2H_5$ |
| $n-C_3H_7$ | $n-C_3H_7$ | $n-C_3H_7$ |

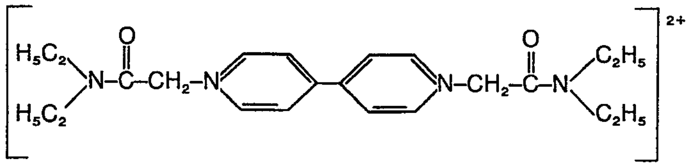

2 Cl⊖

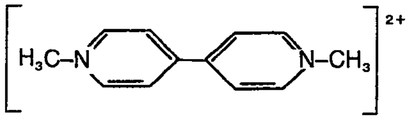

$2CH_3OSO_3^{\ominus}$

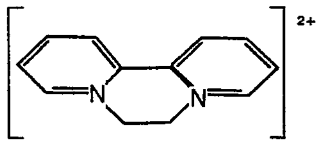

2 Br⊖

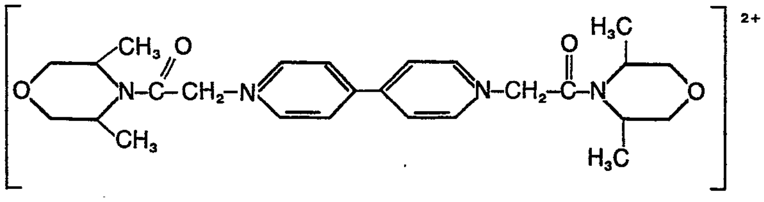

2Cl⊖

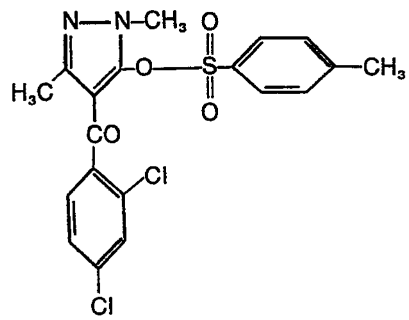

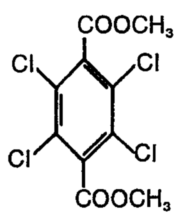

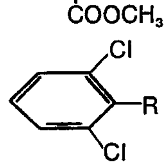

| R |
|---|
| CN |
| $CSNH_2$ |

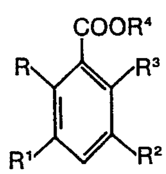

| R | $R^1$ | $R^2$ | $R^3$ | $R^4$ |
|---|---|---|---|---|
| H | Cl | $NH_2$ | Cl | (Salze, Ester, Amide) |
| Cl | Cl | H | Cl | Na |
| H | J | J | J | H |

| R | R¹ | R² | R³ | R⁴ |
|---|---|---|---|---|
| Cl | H | Cl | $OCH_3$ | H |
| Cl | Cl | H | Cl | $(CH_3)_2NH_2$ |

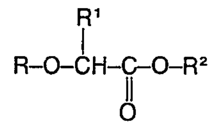

| R | R¹ | R² |
|---|---|---|
| 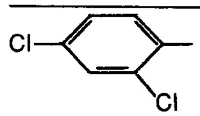 | $CH_3$ | H (Salze, Ester, Amide) |
| 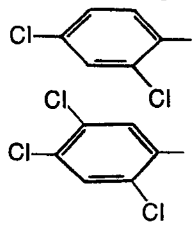 | H | H (Salze, Ester, Amide) |
| | H | H (Salze, Ester, Amide) |
| 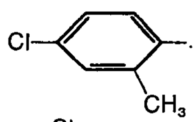 | H | H (Salze, Ester, Amide) |
| 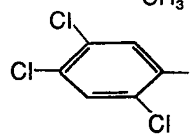 | $CH_3$ | H (Salze, Ester, Amide) |
| 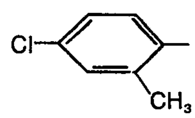 | $CH_3$ | H (Salze, Ester, Amide) |
| | 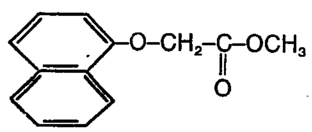 | |
| 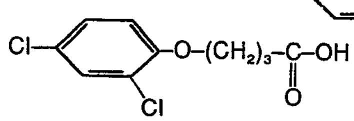 | | (Salze, Ester, Amide) |
| 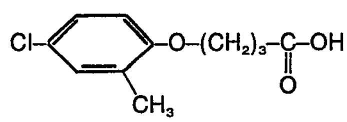 | | (Salze, Ester, Amide) |
| 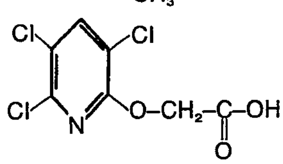 | | (Salze, Ester, Amide) |

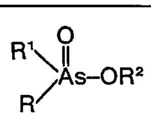

| R | R¹ | R² |
|---|---|---|
| OH | $CH_3$ | Na |
| $CH_3$ | $CH_3$ | Na |
| $CH_3$ | $CH_3$ | OH |
| ONa | $CH_3$ | Na |

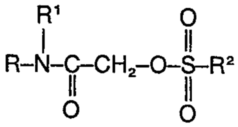

| R | $R^1$ | $R^2$ |
| --- | --- | --- |
| 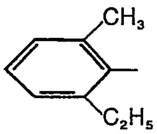 | $-CH_2O-C_3H_5$ | $CH_3$ |
| 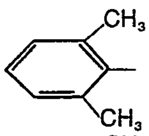 | $i-C_3H_7-O-CH_2-$ | $CH_3$ |
| 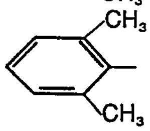 | $-CH_2-O-C_2H_5$ | $CH_3$ |
| 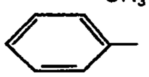 | $i-C_3H_7$ | $-NHCH_3$ |

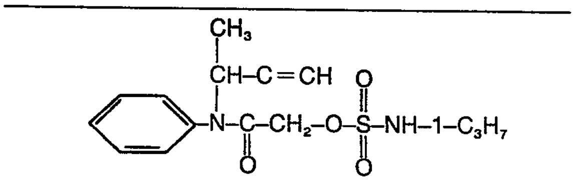

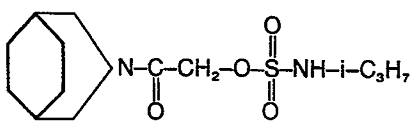

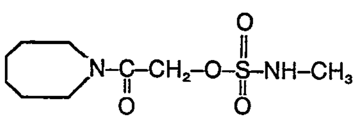

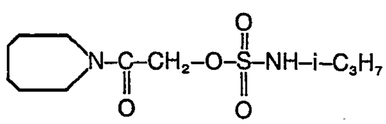

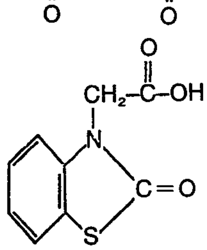

(Salze)

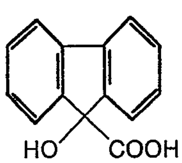

(Salze, Ester)

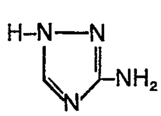

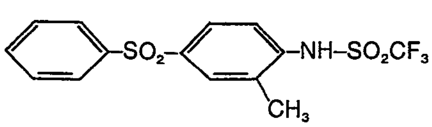

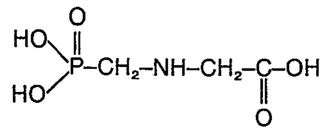

(Salze)

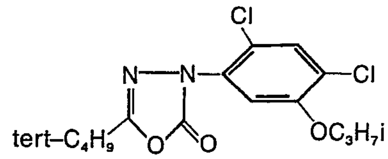

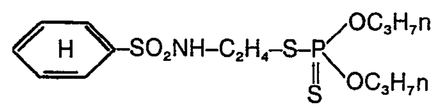

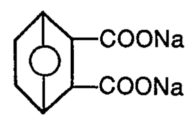

(und andere Salze)

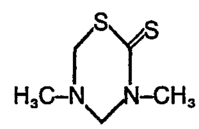

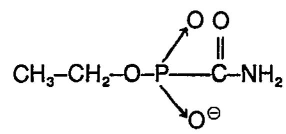

$NH_4^{\oplus}$

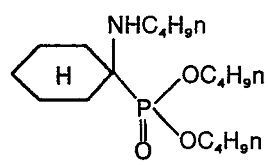

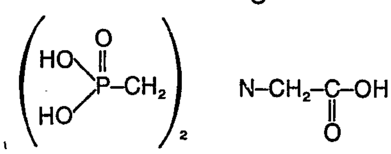

(Salze)

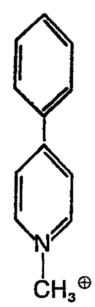

$Cl^{\ominus}$

$NH_4SCN$

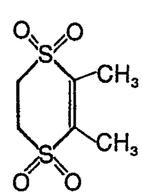

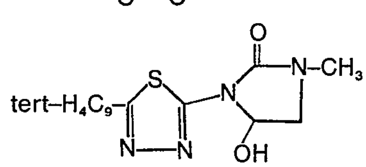

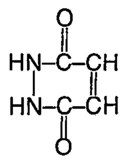

(Salze)

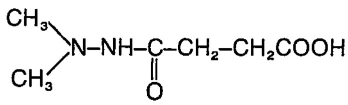

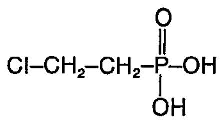

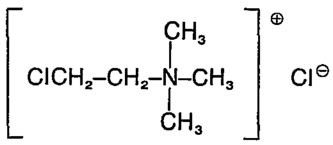

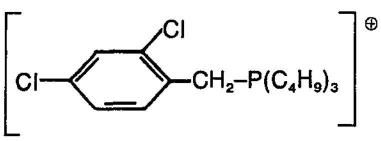

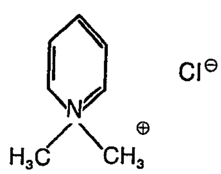

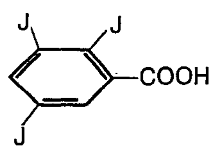

(Salze, Ester, Amide)

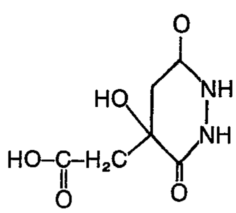

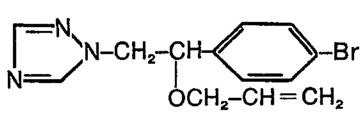

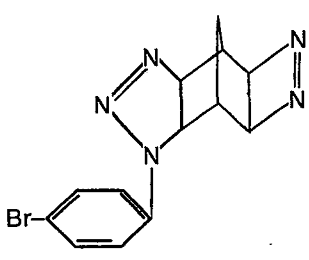

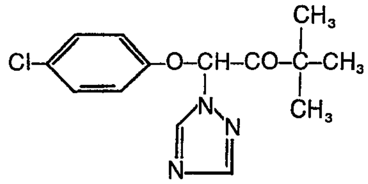

Ausserdem ist es nützlich, die erfindungsgemässen Herbizide allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phythopathogenen Pilzen bzw. Bakterien. Von Interesse sind ferner die Mischbarkeit mit Mineralstofflösungen, welche zur Behebung von Ernährungs- oder Spurenelementmängeln eingesetzt werden. Es kann auch vorteilhaft sein, die erfindungsgemässen Mittel einzeln oder in den möglichen Kombinationen zusammen mit festen oder flüssigen Mineraldüngern gemischt auszubringen.

**Patentansprüche für die Vertragsstaaten: BE – CH – DE – FR – GB – IT – Li – Lv – NL – SE**

1. 4H-3,1-Benzoxazinderivate der Formel I

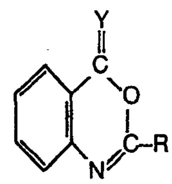

(I),

in der

Y für Sauerstoff oder Schwefel und

R für einen durch Halogenalkoxy, Halogenalkylmercapto oder Halogenalkylsulfonyl mit jeweils 1 bis 4 Kohlenstoffatomen p- oder m-substituierten Phenylrest stehen.

2. Herbizid, enthaltend ein 4H-3,1-Benzoxazinderivat der Formel I gemäss Anspruch 1.

3. Verfahren zur Bekämpfung unerwünschten Pflanzenwuchses, dadurch gekennzeichnet, dass man die Pflanzen oder den Boden mit einem Herbizid, enthaltend ein 4H-3,1-Benzoxazinderivat der Formel I gemäss Anspruch 1, behandelt.

4. Verfahren zur Herstellung von 4H-3,1-Benzoxazinderivaten der Formel I gemäss Anspruch 1, dadurch gekennzeichnet, dass man eine Säure der Formel II

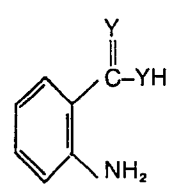

(II),

in der Y die im Anspruch 1 genannten Bedeutungen hat, mit mindestens dem zweifachen molaren Überschuss eines Carbonsäurehalogenids der Formel III

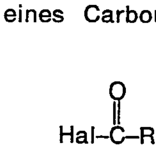

(III),

in der R die im Anspruch 1 genannten Bedeutungen hat und Hal für Halogen steht,

in einem aromatischen tertiären Amin als Lösungsmittel bei einer Temperatur im Bereich zwischen 10 und 60 °C umsetzt.

5. Verfahren zur Herstellung von 4H-3,1-Benzoxazinderivaten der Formel I gemäss Anspruch 1,

dadurch gekennzeichnet, dass man eine Säure der Formel II

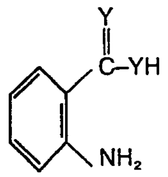

(II),

in der Y die im Anspruch 1 genannten Bedeutungen hat, oder ein Alkalimetall- oder Erdalkalimetallsalz dieser Anthranilsäure mit ungefähr stöchiometrischen Mengen eines Carbonsäurehalogenids der Formel III

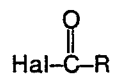

(III),

in der R die im Anspruch 1 genannten Bedeutungen hat und Hal für Halogen steht, in einem inerten organischen Lösungsmittel oder in Wasser und gegebenenfalls in Anwesenheit eines Säureacceptors bei einer Temperatur im Bereich von 0 bis 60 °C zu einem Carbonamid der Formel IV

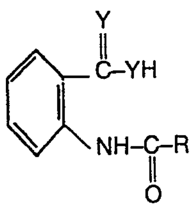

(IV),

in der R und Y die im Anspruch 1 genannten Bedeutungen haben, umsetzt und dieses dann in Gegenwart eines wasserentziehenden Mittels bei einer Temperatur im Bereich zwischen 30 und 150 °C cyclisiert.

**Patentansprüche für den Vertragsstaat AT**

1. Herbizid enthaltend ein 4H-3,1-Benzoxazinderivat der Formel I

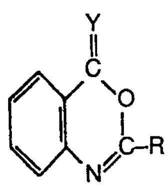

(I),

in der
Y für Sauerstoff oder Schwefel und
R für einen durch Halogenalkoxy, Halogenalkylmercapto, oder Halogenalkylsulfonyl mit jeweils 1 bis 4 Kohlenstoffatomen p- oder m-substituierten Phenylrest stehen.

2. Verfahren zur Herstellung von 4H-3,1-Benzoxazinderivaten der Formel I gemäss Anspruch 1, dadurch gekennzeichnet, dass man eine Säure der Formel II

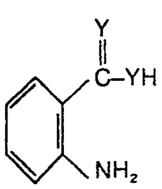

(II),

in der Y die im Anspruch 1 genannten Bedeutungen hat, mit mindestens dem zweifachen molaren Überschuss eines Carbonsäurehalogenids der Formel III

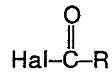

(III),

in der R die im Anspruch 1 genannten Bedeutungen hat und Hal für Halogen steht, in einem aromatischen tertiären Amin als Lösungsmittel bei einer Temperatur im Bereich zwischen 10 und 60 °C umsetzt.

3. Verfahren zur Herstellung von 4H-3,1-Benzoxazinderivaten der Formel I gemäss Anspruch 1, dadurch gekennzeichnet, dass man eine Säure der Formel II

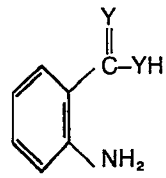

(II),

in der Y die im Anspruch 1 genannten Bedeutungen hat, oder ein Alkalimetall- oder Erdalkalimetallsalz dieser Anthranilsäure mit ungefähr stöchiometrischen Mengen eines Carbonsäurehalogenids der Formel III

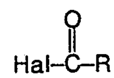

(III),

in der R die im Anspruch 1 genannten Bedeutungen hat und Hal für Halogen steht, in einem inerten organischen Lösungsmittel oder in Wasser und gegebenenfalls in Anwesenheit eines Säureacceptors bei einer Temperatur im Bereich von 0 bis 60 °C zu einem Carbonamid der Formel IV

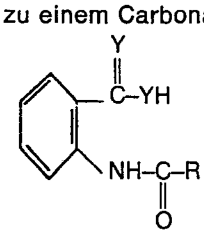

(IV),

in der R und Y die im Anspruch 1 genannten Bedeutungen haben, umsetzt und dieses dann in Gegenwart eines wasserentziehenden Mittels bei einer Temperatur im Bereich zwischen 30 und 150 °C cyclisiert.

**Claims for the contracting states: BE – CH – DE – FR – GB – IT – LI – LU – NL – SE**

1. 4H-3,1-Benzoxazine derivatives of the formula I

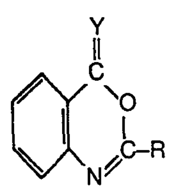

(I),

where
Y is oxygen or sulfur, and

R is phenyl substituted in the p- or m-position by haloalkoxy, haloalkylmercapto or haloalkylsulfonyl, each having 1 to 4 carbon atoms.

2. A herbicide containing a 4H-3,1-benzoxazine derivative of the formula I as claimed in claim 1.

3. A process for controlling unwanted plant growth, wherein the plants or the soil are treated with a herbicide containing a 4H-3,1-benzoxazine derivative of the formula I as claimed in claim 1.

4. A process for the preparation of 4H-3,1-benzoxazine derivatives of the formula I as claimed in claim 1, wherein an acid of the formula II

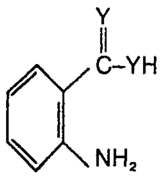

(II),

where Y has the meanings given in claim 1, is reacted with a twofold or even higher molar excess of a carboxylic acid halide of the formula III

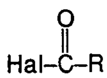

(III),

where R has the meanings given in claim 1, and Hal is halogen, in an aromatic tertiary amine as the solvent, at a temperature of from 10 to 60 °C.

5. A process for the preparation of 4H-3,1-benzoxazine derivatives of the formula I as claimed in claim 1, wherein an acid of the formula II

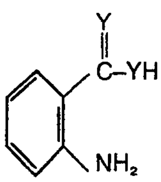

(II),

where Y has the meanings given in claim 1, or an alkali metal salt or alkaline earth metal salt of this anthranilic acid, is reacted with about the stoichiometric amount of a carboxylic acid halide of the formula III

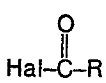

(III),

where R has the meanings given in claim 1, and Hal is halogen, in an inert organic solvent or in water, in the presence or absence of an acid acceptor, at a temperature of from 0 to 60 °C, to give a carboxylic acid amide of the formula IV

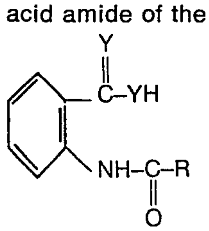

(IV),

where R and Y have the meanings given in claim 1, and this amide is then cyclized at a temperature of from 30 to 150 °C in the presence of a dehydrating agent.

**Claims for the contracting state AT**

1. A herbicide containing a 4H-3,1-benzoxazine derivative of the formula I

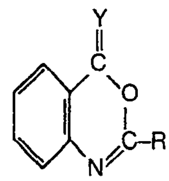

(I),

where

Y is oxygen or sulfur, and

R is phenyl substituted in the p- or m-position by haloalkoxy, haloalkylmercapto or haloalkylsulfonyl, each having 1 to 4 carbon atoms.

2. A process for the preparation of a 4H-3,1-benzoxazine derivative of the formula I as defined in claim 1, wherein an acid of the formula II

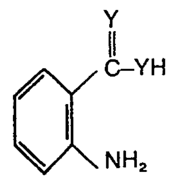

(II),

where Y has the meanings given in claim 1, is reacted with a twofold or even higher molar excess of a carboxylic acid halide of the formula III

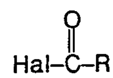

(III),

where R has the meanings given in claim 1, and Hal is halogen, in an aromatic tertiary amine as the solvent, at a temperature of from 10 to 60 °C.

3. A process for the preparation of 4H-3,1-benzoxazine derivatives of the formula I as defined in claim 1, wherein an acid of the formula II

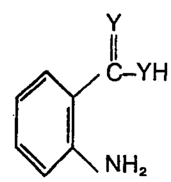

(II),

where Y has the meanings given in claim 1, or an alkali metal salt or alkaline earth metal salt of this anthranilic acid, is reacted with about the stoichiometric amount of a carboxylic acid halide of the formula III

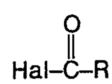

(III),

where R has the meanings given in claim 1, and Hal is halogen, in an inert organic solvent or in water, in the presence or absence of an acid acceptor, at a temperature of from 0 to 60 °C, to give a carboxylic acid amide of the formula IV

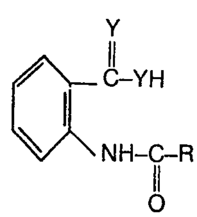

(IV),

where R and Y have the meanings given in claim 1, and this amide is then cyclized at a temperature of from 30 to 150 °C in the presence of a dehydrating agent.

**Revendications pour les Etats contractants: BE – CH – DE – FR – GB – IT – LI – LU – NL – SE**

1. Dérivés de 4H-3,1-benzoxazine de formule I:

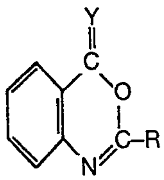

(I),

dans laquelle
Y représente oxygène ou soufre et
R un reste phényle substitué en p- ou m- par halogénalcoxy, halogénalkylmercapto ou halogénalkylsulfonyle, chacun à 1 à 4 atomes de carbone.

2. Herbicide contenant un dérivé de 4H-3,1-benzoxazine de formule 1 selon la revendication 1.

3. Procédé de lutte contre une croissance des plantes indésirable, caractérisé par le fait qu'on traite les plantes ou le sol avec un herbicide contenant un dérivé de 4H-3,1-benzoxazine de formule 1 selon la revendication 1.

4. Procédé de préparation de dérivés de 4H-3,1-benzoxazine de formule 1 selon la revendication 1, caractérisé par le fait qu'on fait réagir un acide de formule II

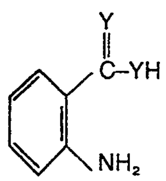

(II),

dans laquelle Y a la signification donnée dans la revendication 1, avec un excès molaire au moins double d'un halogénure d'acide carboxylique de formule III

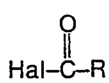

(III),

dans laquelle R a la signification donnée dans la revendication 1 et Hal représente un halogène, dans une amine tertiaire aromatique comme solvant, à une température comprise entre 10 et 60 °C.

5. Procédé de préparation de dérivés de 4H-3,1-benzoxazine de formule 1 selon la revendication 1, caractérisé par le fait qu'on fait réagir un acide de formule II

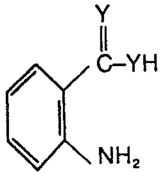

(II),

dans laquelle Y a la signification donnée dans la revendication 1, ou un sel de métal alcalin ou de métal alcalino-terreux de cet acide anthranilique, avec une quantité à peu près stoechiométrique d'un halogénure d'acide carboxylique de formule III

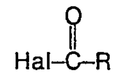

(III),

dans laquelle R a la signification donnée dans la revendication 1 et Hal représente un halogène, dans un solvant inerte ou dans l'eau et éventuellement en présence d'un accepteur d'acide, à une température comprise entre 0 et 60 °C, pour obtenir un carbonamide de formule IV

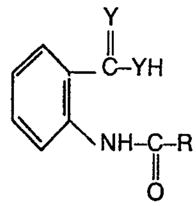

(IV),

dans laquelle R et Y ont les significations données dans la revendication 1 et on cyclise alors celui-ci, en présence d'un agent deshydratant, à une température comprise entre 30 et 150 °C.

**Revendications pour l'Etat contractant AT**

1. Herbicide contenant un dérivé de 4H-3,1-benzoxazine de formule I

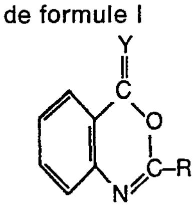

(I),

dans laquelle
Y représente oxygène ou soufre et
R un reste phényle substitué en p- ou m- par halogénalcoxy, halogénalkylmercapto ou halogénalkylsulfonyle chacun à 1 à 4 atomes de carbone.

2. Procédé de préparation de dérivés de 4H-3,1-benzoxazine de formule 1 selon la revendication 1, caractérisé par le fait qu'on fait réagir un acide de formule II

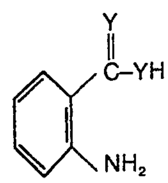

(II),

dans laquelle Y a la signification donnée dans la revendication 1, avec un excès molaire au moins double d'un halogénure d'acide carboxylique de formule III

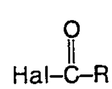

(III),

dans laquelle R a la signification donnée dans la revendication 1 et Hal représente un halogène, dans une amine tertiaire aromatique comme solvant, à une température comprise entre 10 et 60 °C.

3. Procédé de préparation de dérivés de 4H-3,1-benzoxazine de formule 1 selon la revendication 1, caractérisé par le fait qu'on fait réagir un acide de formule II

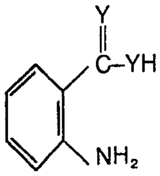

(II),

dans laquelle Y a la signification donnée dans la revendication 1, ou un sel de métal alcalin ou de métal alcalino-terreux de cet acide anthranilique, avec une quantité à peu près stoechiométrique d'un halogénure d'acide carboxylique de formule

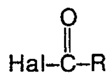

(III),

dans laquelle R a la signification donnée dans la revendication 1 et Hal représente un halogène, dans un solvant inerte ou dans l'eau et éventuellement en présence d'un accepteur d'acide, à une température comprise entre 0 et 60 °C, pour obtenir un carbonamide de formule IV

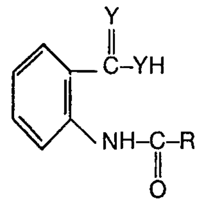

(IV),

dans laquelle R et Y ont les significations données dans la revendication 1 et on cyclise alors celui-ci en présence d'un agent deshydratant à une température comprise entre 30 et 150 °C.